# EUROPEAN PATENT SPECIFICATION

(11) **EP 4 676 354 B1**
(45) Date of publication and mention of the grant of the patent: **02.12.2026**
(21) Application number: 24712903.4
(22) Date of filing: 07.03.2024
(51) Int. Cl.: A61B 17/16, A61B 17/92

(54) **ORTHOPAEDIC IMPACTOR**
ORTHOPÄDISCHER IMPAKTOR
IMPACTEUR ORTHOPÉDIQUE

(30) Priority: 07.03.2023 GB 202303320
(43) Date of publication of application: 14.01.2026
(73) Proprietor: De Soutter Medical Limited, Aylesbury, Buckinghamshire HP22 5WF (GB)
(72) Inventor: DESOUTTER, George, Aylesbury Buckinghamshire HP22 5WF (GB); KEENS, Matthew, Aylesbury Buckinghamshire HP22 5WF (GB); GOOCH, Matthew, Aylesbury Buckinghamshire HP22 5WF (GB)
(74) Representative: Marks & Clerk LLP
(86) International application number: PCT/GB2024/050604
(87) International publication number: WO 2024/184647

(56) References cited:
- WO-A1-2018/044348
- DE-A1- 102010 017 726

## Description

### TECHNICAL FIELD

The present disclosure relates to electromagnetic surgical impactors. In particular, but without limitation, this disclosure relates to electromagnetically driven orthopaedic impactors.

### BACKGROUND

When performing surgery, particularly in orthopaedic surgery, it is sometimes necessary to provide an impact force. This can be through the use of a surgical mallet or slap hammer. For instance, in total hip arthroplasty, a surgeon may utilise a broach tool to prepare a femur to receive a hip implant (femoral broaching). A surgical mallet may be utilised to force the broach into the femur. The use of surgical mallets or slap hammers can be imprecise and strenuous on the surgeon.

DE 10 2010 017726 A1 proposes a tool having a housing and a percussion anvil/striking mechanism arrangement reciprocatively driven by a driving device along an axis in a forward direction or a rearward direction. The device is formed by a magnet, an induction coil, and a spring that sets a striking mechanism into a resting position.

### SUMMARY

The present disclosure relates to electromagnetic surgical impactors, and in particular, to electromagnetic surgical impactors having an improved centring system for providing increased impact force.

According to a first aspect there is provided an electromagnetic surgical impactor comprising: a housing forming an internal cavity extending along a longitudinal axis; a striker located within the internal cavity and configured to move along the longitudinal axis; an anvil system comprising a first anvil wherein the anvil system is configured to move in a first direction along the longitudinal axis from a first resting position towards a first end point, an electromagnetic drive system configured to drive the striker to move in the first direction to impact the first anvil such that a first force directed in the first direction is imparted to an external object via the first anvil; and a centring system configured to bias the first anvil towards the first resting position. The centring system comprises: one or more first resilient members configured to bias the first anvil towards the first resting position in response to the first anvil being displaced towards the first end point, beyond a first engagement position; and one or more second resilient members configured to bias the first anvil towards the first resting position in response to the first anvil being displaced towards the first end point, beyond a second engagement position, wherein the second engagement position is located between the first engagement position and the first end point. Each of the first resilient members has a lower stiffness than each of the second resilient members

In certain embodiments, two types of resilient members are provided. The first resilient member(s) have a lower stiffness and engage at a lower displacement of the anvil system than the second resilient member(s). This helps centre the anvil system whilst reducing any negative affect by the centring system on the impact force provided by the impactor.

In one embodiment, the one or more first resilient members are configured to bias the first anvil towards the first resting position over a first range of motion of the first anvil that encompasses at least a portion of a range between the second engagement position and the first resting position. The first range of motion may fully encompass the range between the second engagement position and the first resting position. The first range of motion may be a range from the first end point to the first resting position. The first engagement position may be the first resting position.

In one embodiment, the one or more second resilient members are configured to bias the first anvil towards the first resting position over a second range of motion of the first anvil, wherein the second range of motion extends over at least a portion of a range between the second engagement position and the first end point. The second range of motion may fully encompass the range between the second engagement position and the first end point. The second range of motion may be a range from the first end point to the second engagement position.

The one or more second resilient members may provide a stronger biasing force per unit distance of movement of the anvil system than the one or more first resilient members. The first and second resilient members may be considered biasing members.

In one embodiment, the surgical impactor is configured such that the first anvil may not move beyond the first end point. The first end point may be a position or point defining an end of a full range of motion of the first anvil.

According to an embodiment, the first and second resilient members are springs, wherein each second resilient member has a higher spring constant than each first resilient member. Conversely, each first resilient member may have a lower spring constant than each second resilient member.

According to an embodiment, the one or more first resilient members comprise one or more coil springs and/or the one or more second resilient members comprise one or more leaf springs.

According to an embodiment, centring system further includes one or more third resilient members configured to bias the first anvil towards the first resting position in response to the first anvil being displaced towards the first end point, beyond a third engagement position, wherein the third engagement position is located between the second engagement position and the first end point.

The one or more third resilient members may be configured to bias the first anvil towards the first resting position over a third range of motion of the first anvil that includes at least a portion of the range from the first end point to the third engagement position. The first end point and the third engagement position may both be located further from the first resting position in the first direction than the second engagement position. The third range of motion may fall within the first range of motion and/or within the second range of motion. The third range of motion may fall outside one or both of the first and second ranges of motion. In one embodiment, the third range of motion is a range between the first end point and the third engagement position. The one or more third resilient members may be configured to prevent the first anvil from passing beyond the first end point.

The one or more third resilient members may have a stiffness that is greater than that of the one or more first resilient members and/or the one or more second resilient members. Having said this, this is not essential. Accordingly, the one or more third resilient members may have a stiffness that is less than or equal to that of the one or more first resilient members and/or the one or more second resilient members.

According to an embodiment, the one or more third resilient members comprises one or more dampers, each comprising a shock absorbent material. The shock absorbent material may be silicone. The one or more dampers may be silicone dampers. The one or more third resilient members may be configured to dampen movement of the anvil beyond the third engagement position (in the first direction).

According to an embodiment, the centring system is configured to prevent the striker travelling beyond the first end point.

According to an embodiment, the anvil system comprises a second anvil connected to the first anvil, wherein the second anvil is configured to move in a second direction, opposite to the first direction, from a second resting position to a second end point, wherein the first and second anvils are positioned on opposite sides of the striker; and the electromagnetic drive system is further configured to drive the striker to move in the second direction to impact the second anvil such that a second force directed in the second direction is imparted to the external object via the second anvil. According to this embodiment, the centring system further comprises: one or more fourth resilient members configured to bias the second anvil towards the second resting position in response to the second anvil being displaced towards the second end point, beyond a fourth engagement position, and one or more fifth resilient members configured to bias the second anvil towards the second resting position in response to the second anvil being displaced towards the second end point, beyond a fifth engagement position, wherein the fifth engagement position is located between the fourth engagement position and the second end point, wherein each of the fourth resilient members has a lower stiffness than each of the fifth resilient members.

In one embodiment, the one or more fourth resilient members are configured to bias the second anvil towards the second resting position over a fourth range of motion of the second anvil that encompasses at least a portion of a range between the forth engagement position and the second resting position. The fourth range of motion may fully encompass the range between the fourth engagement position and the second resting position. The fourth range of motion may be a range from the second end point to the second resting position. The fourth engagement position may be the second resting position.

In one embodiment, the one or more fifth resilient members are configured to bias the second anvil towards the second resting position over a fifth range of motion of the second anvil, wherein the fifth range of motion extends over at least a portion of a range between the fifth engagement position and the second end point. The fifth range of motion may fully encompass the range between the fifth engagement position and the second end point. The fifth range of motion may be a range from the second end point to the fifth engagement position.

The one or more fifth resilient members may provide a stronger biasing force per unit distance of movement of the anvil system than the one or more fourth resilient members. The fourth and fifth resilient members may be considered biasing members.

In one embodiment, the surgical impactor is configured such that the second anvil may not move beyond the second end point. The second end point may be a position or point defining an end of a full range of motion of the second anvil. The anvil system may have a full range of motion between the first end point and the second end point.

According to an embodiment, the fourth and fifth resilient members are springs, wherein each fifth resilient member has a higher spring constant than each fourth resilient member.

According to an embodiment, the one or more fourth resilient members comprise one or more coil springs and/or wherein the one or more fifth resilient members comprise one or more leaf springs.

According to an embodiment, the centring system further includes one or more sixth resilient members configured to bias the second anvil towards the second resting position in response to the second anvil being displaced towards the second end point, beyond a sixth engagement position, wherein the sixth engagement position is located between the fifth engagement position and the second end point.

The one or more sixth resilient members may be configured to bias the second anvil towards the second resting position over a sixth range of motion of the second anvil that extends over at least a portion of a range between the second end point and the fifth engagement position. The second end point and the sixth engagement position may both be located further from the second resting position in the second direction than the fifth engagement position. The sixth range of motion may fall within the fourth range of motion and/or within the fifth range of motion. The sixth range of motion may fall outside one or both of the fourth and fifth ranges of motion. In one embodiment, the sixth range of motion is a range between the second end point and the sixth engagement position.

The one or more sixth resilient members may have a stiffness that is greater than that of the one or more fourth resilient members and/or the one or more fifth resilient members. Having said this, this is not essential. Accordingly, the one or more sixth resilient members may have a stiffness that is less than or equal to that of the one or more fourth resilient members and/or the one or more fifth resilient members.

The one or more sixth resilient members may be configured to prevent the second anvil from passing beyond the second end point.

According to an embodiment, the one or more sixth resilient members comprises one or more dampers, each comprising a shock absorbent material. The shock absorbent material may be silicone. The one or more dampers may be silicone dampers. The one or more sixth resilient members may be configured to dampen movement of the anvil beyond the sixth engagement position (in the second direction).

According to an embodiment, the centring system is configured to prevent the striker travelling beyond the second end point.

According to an embodiment, the separation between the first and second anvils in the first direction is less than a maximum range of motion over which the electromagnetic drive system is configured to drive the striker.

According to an embodiment, the electromagnetic surgical impactor further comprises circuitry configured to control the electromagnetic drive system to drive the striker according to a mode of operation. The mode of operation may be selectable between: a first mode of operation that controls the electromagnetic drive system to drive the striker in the first direction to impact the first anvil to impart a primary force in the first direction; a second mode of operation that controls the electromagnetic drive system to drive the striker in the second direction to impact the second anvil to impart a primary force in the second direction; a reciprocating mode of operation that controls the electromagnetic drive system to alternately drive the striker in the first direction and in the second direction to alternately impact the first anvil to impart a primary force in the first direction and impact the second anvil to impart a primary force in the second direction.

Whilst the first and second modes of operation drive the striker to impart primary forces in the first and second directions respectively, they also may drive the striker to move opposite to these directions (e.g. to prepare to be driven). When preparing to be driven, the striker may impart a secondary force to the anvil system in an opposite direction to the primary force in that mode of operation. This secondary force may be smaller (e.g. may have a smaller magnitude) than the primary force.

According to an embodiment, the first anvil is connected to the second anvil via a rod which passes through a channel in the striker. The rod may be a central rod that passes through a central channel in the striker (e.g. along the longitudinal axis). The striker may be configured to move (e.g. slide) along the rod.

According to an embodiment, at least one of the one or more first resilient members and the one or more second resilient members is configured to impart a first biasing force on the second anvil in the second direction to impart tension on the anvil system when biasing the first anvil towards the first resting position. In addition, or alternatively, at least one of the one or more fourth resilient members and the one or more fifth resilient members may be configured to impart a second biasing force on the first anvil in the first direction to impart tension on the anvil system when biasing the second anvil towards the second resting position.

By imparting tension, the biasing force(s) may stretch (rather than compresses) the connection (e.g. the rod) between the first and second anvil. This can be achieved applying the biasing force on the opposite anvil to the one that was impacted. This can help to reduce the risk of the anvil system buckling during use. In a specific embodiment, the one or more second resilient members are configured to impart the first biasing force on the second anvil in the second direction and the one or more fifth resilient members impart the second biasing force on the first anvil in the first direction.

According to an embodiment, the centring system is configured to impart a biasing force to the striker via the anvil system to bias the striker towards a central position after each impact between the striker and the anvil system. In a specific embodiment, the centring system does not act directly on the striker, but instead acts via the anvil system. The striker may not be directly biased towards the central position. The central position may be a position where the striker is separated from the anvil system when the striker and anvil system are at rest. The central position may be a resting position of the striker.

According to an embodiment, the electromagnetic surgical impactor further comprises a tool or tool holder connected to the second anvil for imparting force from the second anvil to the external object. The tool may be hammer, broach, puller/extractor (e.g. implant extractor), etc.

According to an embodiment, the centring system is configured to allow the anvil system to float within the housing to allow a user to alter an impact force between the striker and the anvil system through pushing or pulling the electromagnetic surgical impactor to adjust a position of the anvil system relative to the striker. This may allow the resting position of the anvil system (e.g. the first resting position and the second resting position) to be adjusted.

### BRIEF DESCRIPTION OF THE DRAWINGS

Arrangements of the present invention will be understood and appreciated more fully from the following detailed description, made by way of example only and taken in conjunction with drawings in which:
FIG. 1 shows a side view of an electromagnetic impactor according to an embodiment;
FIG. 2 shows a cross-section of a driving mechanism for the electromagnetic impactor;
FIG. 3 shows a zoomed in cross-section of a front end of the driving mechanism;
FIG. 4 shows a zoomed in cross-section of a rear end of the driving mechanism; and
FIG. 5 shows a zoomed in cross-section of a central portion of the driving mechanism.

### DETAILED DESCRIPTION

This application relates to surgical impactors for use in imparting an impact force (e.g. as a replacement to traditional hammers and mallets). In particular, the application relates to electromagnetically driven surgical impactors.

A surgical impactor may be driven by accelerating a striker (or strike assembly) to impact an anvil that is connected to a tool for conveying the force to an external object. Whilst impactor devices can be driven pneumatically, such pneumatic devices can be large and unwieldy. By making use of an electromagnetic drive system, the impactor system can be made smaller and easier to use.

As the striker is driven linearly, a rectilinear motor may be utilised to drive the striker. Using a rectilinear motor avoids the need to convert rotational motion to linear motion (e.g. through gears). An issue with rectilinear motors is that they can become jammed if the motor armature (in this case, the striker) overextends (overtravel cogging). Accordingly, it can be advantageous to provide a centring system (e.g. in the form of one or more resilient members, such as one or more springs) to help return the striker to a central position to enable it to be driven again. This can allow the striker to over travel, thereby providing additional force, without causing the rectilinear motor to jam through overtravel cogging.

Having said the above, biasing the striker towards a central position will reduce the impact force of the striker, by opposing the driving force provided by the motor. Embodiments described herein provide a centring system that acts upon the anvil to avoid reducing the initial impact force of the striker. After impact, the striker can be urged back towards a central position through the centring system urging the anvil against the striker.

Furthermore, in the embodiments described herein the centring system is provided with two sets of resilient members with differing stiffness. The stiffer resilient member(s) engage at a further point of displacement of the anvil than the less stiff resilient members. The less stiff resilient member(s) provide a centring force to help position the anvil and striker at respective resting positions without overly limiting the impact force. The stiffer resilient member(s) help to stop the anvil (and striker) towards an end point of travel before urging the anvil (and striker) back towards a more central position. The stiffer resilient member(s), through interaction with the anvil, help return the anvil to a more central position to allow the striker to be driven by the motor. This allows the striker to move outside of a normal operating range of motion of the motor whilst still being returned to this operating range by the centring system. This therefore allows the striker to be driven to impact the anvil at a faster speed without risking overtravel cogging. As the stiffer resilient member(s) engage at a greater displacement than the less stiff resilient member(s) (e.g. as there is a range of motion of the anvil over which the stiffer resilient member(s) do not act), a greater amount of the impact force is imparted to the external object.

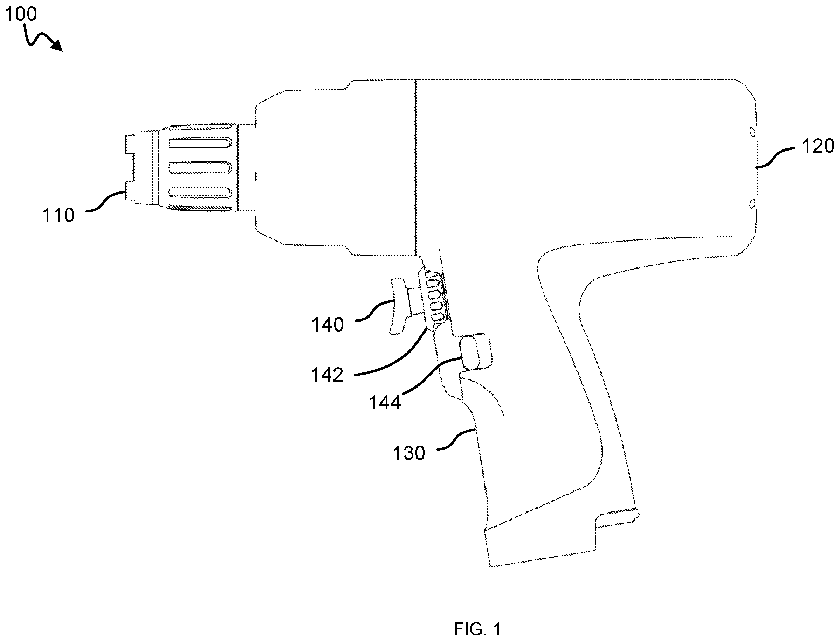

FIG. 1 shows a side view of an electromagnetic impactor 100 according to an embodiment. The impactor 100 includes a tool holder 110, a housing 120 and a handle 130.

The tool holder 110 is configured to releasably secure and drive a tool (not shown). A coupling mechanism 112 is provided in the tool holder 110 for securing a tool within the tool holder 110. For instance, a broaching tool, a hammer tool or a pulling tool (e.g. an implant extractor) may be secured in the tool holder 110 and driven through movement of the tool holder 110. As an alternative to including a tool holder 110, the impactor 100 may instead include an integrated tool. For convenience of description, the remainder of the application is written based on the impactor 100 having a tool holder 110.

A driving mechanism (not shown) for driving the tool holder 110 to move is contained within the housing 120. The driving mechanism may comprise a rectilinear motor configured to drive a striker to impact an anvil system connected to the tool holder 110. The driving mechanism may be configured to drive the striker along a longitudinal axis. The driving mechanism shall be discussed in more detail below. The driving mechanism may be controlled through processing circuitry. The processing circuitry may be contained within the housing 120 (e.g. within the handle 130).

The handle 130 may be shaped to be held in one hand. The handle includes a trigger 140, a power control dial 142 and a mode switch 144. The trigger 140, power control dial 142 and mode switch 144 may provide respective inputs to the processing circuitry.

The trigger 140 provides an input for controlling the activation of the impactor 100. When the trigger 140 is depressed, the tool holder 110 is driven.

The power control dial 142 provides an input for controlling an impact force of the impactor 100. The impact force can be increased or decreased in response to input from the power control dial 142. Based on this input, the processing circuitry can adjust a speed at which the striker is driven by the rectilinear motor.

The mode switch 144 provides an input for switching between different modes of operation. The impactor 100 may be configured to operate in three different modes: forward mode, reverse mode, and reciprocating mode (which may otherwise be known as oscillating mode).

In forward mode, the impactor 100 provides a primary force in a forward direction (a first direction along the longitudinal axis). In this mode, the striker is driven to impact a front anvil within the anvil system. The forward mode can provide an impact, or pushing, force.

In reverse mode, the impactor 100 provides a primary force in a rearward direction (a second direction along the longitudinal axis), opposite to the forward direction. In this mode, the striker is driven to impact a rear anvil within the anvil system. The reverse mode can provide a removal, or pulling, force.

In reciprocating model, the impactor 100 reciprocates between forward and reverse forces. The striker is alternately driven to impact the front anvil to impart a forward force, and to impact the rear anvil to impart a rearward force. The specific processes involved in each mode will be described in more detail below.

Whilst the present embodiment is described as having a "trigger", a "power control dial" and a "mode switch", it will be appreciated that the functions of each of these input components may be implemented through a variety of means. For instance, these inputs may be in the form of any of, or a combination of, a trigger, dial, switch, slider, touch screen, or any other form of input device.

The impactor 100 is provided with a power source. This may either be in the form of a wired connection (e.g. to a mains power supply) or a connection to a battery. For instance, a battery dock may be provided at a bottom end of the handle 130. A battery may be secured in the battery dock to provide power to the impactor 100.

In specific embodiments the electromagnetic impactor 100 is a surgical impactor. In this case, the electromagnetic impactor 100 is suitable for use in a surgical setting. This may require the use of sterilisable materials. In addition, or alternatively, this may require the electromagnetic impactor 100 being waterproof. For instance, a waterproof housing may be provided. Furthermore, the electromagnetic impactor 100 may be made from materials suitable for reprocessing (sterilisation and disinfection processes). Whilst the electromagnetic impactor 100 itself may be suitable for reprocessing, tools (e.g. broach tools, hammer tools, puller tools, etc.) for fitting within the device may be replaceable.

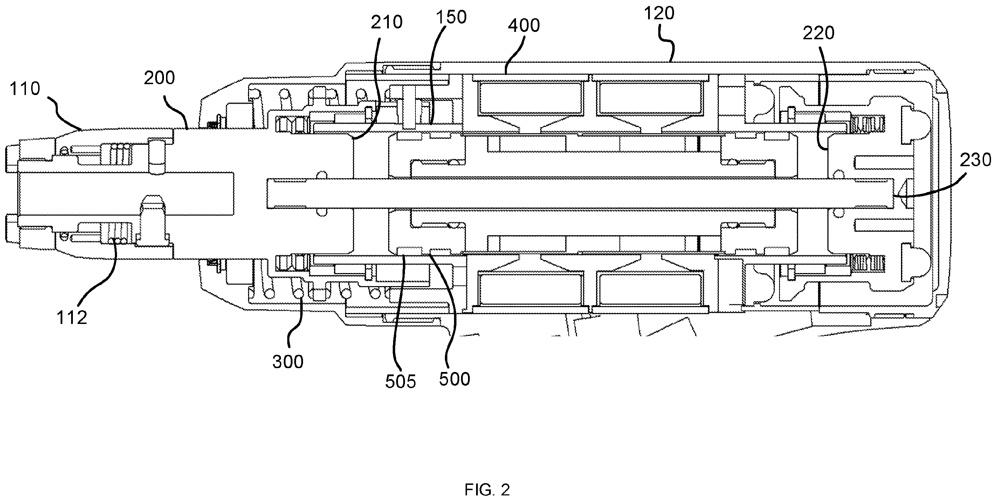

FIG. 2 shows a cross-section of a driving mechanism for the electromagnetic impactor.

The driving mechanism includes an anvil system 200, a centring system 300, a motor 400 and a striker 500. The system shown in FIG. 2 is shown with the anvil system 200 in a resting position where no external forces are acting on the anvil system 200 and the motor 400 is not engaged (e.g. where the centring system 300 and anvil system 200 are in equilibrium). The striker 500 is similarly shown in a corresponding resting position which may be a central position within the motor 400.

The anvil system 200 includes a front anvil 210, a rear anvil 220 and a connector 230. The front anvil 210 is connected to (e.g. integrated with) the tool holder 110, such that movement of the anvil system 200 causes the tool holder 110 to also move. The front anvil 210 and the rear anvil 220 are positioned on either side of the striker 500. The connector 230 connects the front anvil 210 to the rear anvil 220 such that the anvil system 200 forms a single unit. In the present embodiment, the connector 230 is in the form of a shaft that runs along a longitudinal axis of the impactor 100.

The striker 500 is mounted on the connector 230 such that is may slide along the longitudinal axis within a cavity within the housing. The striker 500 includes bearings 505 (e.g. linear bearings) which are configured to assist the striker 500 in moving longitudinally. The bearings 505 may act on an internal wall 150 within the impactor 100. The internal wall 150 may define a longitudinal cavity within which the striker 500 is housed. The longitudinal cavity may be cylindrical.

The striker 500 is configured to be driven by the motor 400 to impact the anvil system 200 to impart a force to the tool holder 110. When the striker 500 is driven in a first direction, it impacts the front anvil 210 and imparts a force in the first direction. When the striker 500 is driven in a second direction, it impacts the rear anvil 220 and imparts a force in the second direction. The first and second directions are opposite to each other and are both parallel to the longitudinal axis.

The first direction may be considered a forward direction and the second direction may be considered a rearward direction. The terms "forward" and "rearward" are intended to be relative terms (i.e. relative to the structure of the impactor 100) and are not intended to relate to any orientation of the impactor 100 in use. For the remainder to the description, the terms "forward direction" and "forward" shall be used to refer to the first direction and the terms "rearward direction" and "rearward" shall be used to refer to the second direction.

The centring system 300 comprises a number of resilient members (e.g. springs, dampers, etc.). The centring system 300 is configured to bias the anvil system 200 towards a resting position. For instance, after the anvil system 200 has been driven forward within the housing 120, the centring system 300 is configured to bias the anvil system 200 rearwards, towards the resting position of the anvil system 200. Similarly, after the anvil system 200 has been driven backwards within the housing 120, the centring system 300 is configured to bias the anvil system 200 forwards, towards the resting position of the anvil system.

The biasing forces provided by the centring system 300 help to return the striker 500 to a central position. As the anvil system 200 is returned to its resting position, the anvil system 200 imparts a returning force onto the striker to return the striker 500 to its resting position. This allows the striker 500 to be returned to a central position, suitable for being driven again by the motor 400, without requiring the motor 400 to provide a returning force (although the motor 400 may also provide such a returning force).

By configuring the centring system 300 to act on the anvil system 200, the striker 500 may be returned to a central position without requiring the striker 500 itself to be directly biased. This allows the striker 500 to be accelerated by the motor 400 without any biasing forces resisting the acceleration. In contrast, if the striker 500 were directly biased (e.g. through springs acting directly on the striker 500), then the returning forces acting on the striker 500 would act against the acceleration of the striker 500 and would limit the initial force imparted by the striker 500 to the anvil system 200.

In addition, by providing a centring system 300, the striker 500 may be driven beyond a maximum operation range of the motor 400. For instance, the motor 400 may have a maximum displacement of the striker 500, beyond which the motor 400 is no longer able to effectively drive the striker 500. For instance, a brushless rectilinear motor 400 may be unable to return the motor armature (in this case, the striker 500) if it moves further than the full cogging distance of the motor 400. Beyond this point, the motor 400 may jam due to cogging. Accordingly, by providing a centring system 300 configured to bias the anvil system 200 towards a resting position, the motor 400 may drive the striker 500 beyond the maximum range of operation for the motor 400 whilst still allowing the striker 500 to be returned to a central position within the motor 400 to allow continued operation. This allows the motor 400 to drive the striker 500 at a higher velocity without risking the motor 400 jamming.

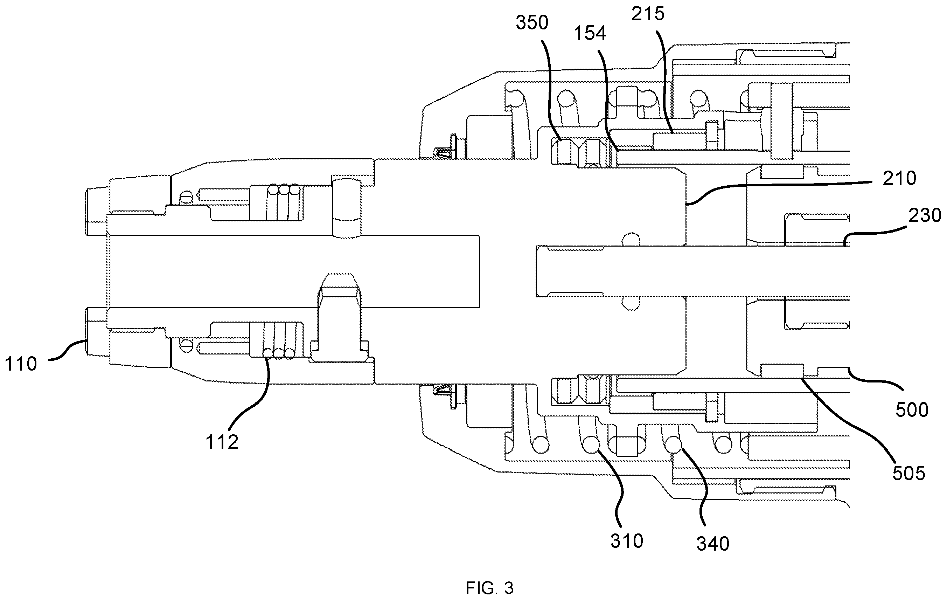

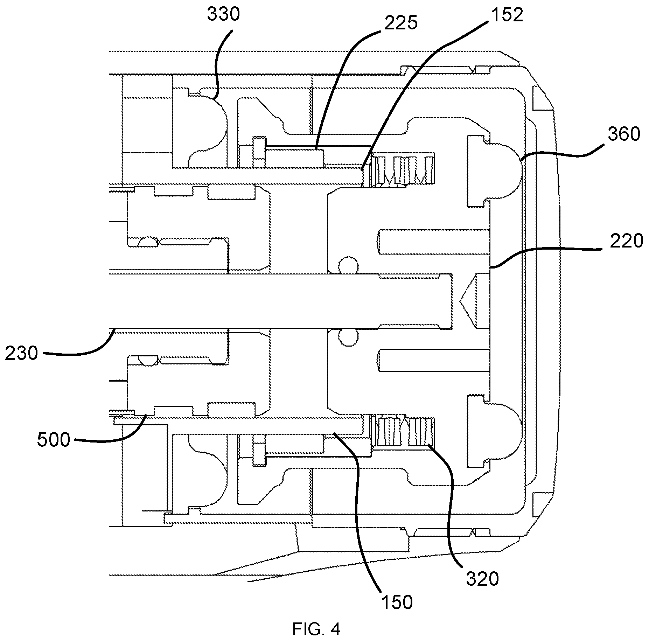

FIG. 3 shows a zoomed in cross-section of a front end of the driving mechanism. FIG. 4 shows a zoomed in cross-section of a rear end of the driving mechanism. The centring system 300 shall be described with reference to both FIG 3 and FIG. 4.

The front anvil 210 is located on a forward side of the striker 500. The rear anvil 220 is located on a rearward side of the striker 500. The anvil system 200 is configured to move forwards and backwards along the longitudinal axis. Bearings 215 (e.g. linear bearings) are provided on the front anvil 210. Similar bearings 225 (e.g. linear bearings) are provided on the rear anvil 220. The bearings 215, 225 on the anvil system 200 are configured to assist the striker anvil system 200 in moving longitudinally. The bearings 215, 225 may act on the internal wall 150 (e.g. one or more external faces of the internal wall 150). The one or more external faces of the internal wall 150 may be cylindrical.

The centring system 300 includes a set of weak springs 310, 340 and a set of stiff springs 320, 350. The stiff springs 320, 350 have a higher stiffness than the weak springs 310, 340. The terms "weak" and "stiff" herein are relative terms, in that they refer to the fact that the stiff springs 320, 350 have a higher stiffness than the weak springs 310, 340. The relative terms "weak" and "stiff" do not imply any particular amount of stiffness, other than this relative stiffness. In the present embodiment, the weak springs 310, 340 are coil springs and the stiff springs 320, 350 are leaf springs, although alternative types of springs (or alternative types of resilient members other than springs) may be utilised.

A resilient member (e.g. a spring) that has a higher "stiffness" may provide a stronger biasing force per distance travelled (e.g. per distance compressed or stretched). For a linear spring, this may be indicated by a higher spring constant, k over a linear region of the stress-strain curve for that spring. Nevertheless, the springs discussed herein need not be linear springs, but could equally be non-linear springs. When comparing the stiffness of two springs, the same range of motion (e.g. of compression or stretching) may be utilised. The stiffness of the springs may be compared using similar environmental parameters (e.g. at the same temperature, e.g. 20°C).

In the present embodiment, the weak springs 310, 340 are located at the front of the impactor 100 and bias the anvil system 200 towards the resting position through interaction with the front anvil 210. A first weak spring 310 provides a biasing force in a rearward direction in response to the first anvil 210 moving forwards beyond the resting position. A second weak spring 340 provides a biasing force in a forward direction in response to the first anvil 210 moving backwards beyond the resting position. Whilst the first weak spring 310 and the second weak spring 340 are provided at the front of the impactor 100 and act on the front anvil 210, this is not essential. The position of the weak springs 310, 340 may be varied, and they may act on various sections of the anvil system 200 (e.g. positioned towards the rear of the impactor 100 and acting on the rear anvil 220).

The first weak spring 310 (e.g. a set of one or more first resilient members) biases the first anvil 210 in response to the first anvil 210 being displaced forwards beyond a first weak spring engagement position (e.g. a first engagement position). In the present embodiment, the first weak spring engagement position is the same as the resting position of the first anvil 210. Having said this, the first weak spring engagement position may be located between the resting position of the first anvil 210 and the first end point. In the present embodiment, the first weak spring 310 biases the first anvil 210 over a range of motion from the resting position of the first anvil 210 to the first end point. The first end point defines a limit in the forward direction beyond which the anvil system 200 may not pass.

The second weak spring 340 (e.g. a set of one or more fourth resilient members) biases the second anvil 220 in response to the second anvil 220 being displaced rearwards beyond a second weak spring engagement position (e.g. a fourth engagement position). In the present embodiment, the second weak spring engagement position is the same as the resting position of the second anvil 220. Having said this, the second weak spring engagement position may be located between the resting position of the second anvil 210 and the second end point. In the present embodiment, the second weak spring 340 biases the second anvil 220 over a range of motion from the resting position of the second anvil 220 to the second end point. The second end point defines a limit in the reward direction beyond which the anvil system 200 may not pass.

In the present embodiment a first set of stiff springs 320 (e.g. a set of one or more second resilient members) are provided at the rear of the impactor 100 and a second set of stiff springs 350 (e.g. a set of one or more fifth resilient members) are provided at the front of the impactor 100.

The first set of stiff springs 320 bias the anvil system 200 towards the resting position (e.g. bias the front anvil 210 towards the resting position for the front anvil 210) through interaction with the rear anvil 220. In the present embodiment, the first set of stiff springs 320 are provided in the rear anvil 220. The first set of stiff springs 320 bias the rear anvil 220 against rearward facing abutments 152 connected to the housing 120. The first set of stiff springs 320 provide a rearward biasing force. The rearward facing abutments 152 may be rear faces of the internal wall 150.

The second set of stiff springs 350 bias the anvil system 200 towards the resting position (e.g. bias the rear anvil 220 towards the resting position for the rear anvil 220) through interaction with the front anvil 210. In the present embodiment, the second set of stiff springs 350 are provided in the front anvil 210. The second set of stiff springs 320 bias the front anvil 210 against forward facing abutments 154 connected to the housing 120. The second set of stiff springs 350 provide a forward biasing force. The forward facing abutments 154 may be forward faces of the internal wall 150.

By providing a rearward biasing force that acts on the rear anvil 220, and a forward biasing force that acts on the front anvil 210, the stiff springs 320, 350 act to apply tension to stretch the anvil system 200 when arresting the motion of the anvil system 200. This protects the connector 230 from buckling.

When the anvil system 200 is at its resting position (when the first anvil 210 and the second anvil 220 are at their respective resting positions), a gap is provided between the stiff springs 320, 350 and their respective abutments 152, 154. There is therefore a central range of motion of the anvil system 200 over which the stiff springs 320, 350 do not engage with the anvil system 200.

The first set of stiff springs 320 engage with the anvil system 200 once the anvil system 200 has moved forward beyond a first stiff spring engagement position (e.g. a second engagement position). The distance between the first stiff spring engagement position and the resting position is equal to the gap between first set of stiff springs 320 and the rearward facing abutment 152 when the anvil system 200 is in the resting position.

The second set of stiff springs 350 engage with the anvil system 200 once the anvil system 200 has moved backwards beyond a second stiff spring engagement position (e.g. a fifth engagement position). The distance between the second stiff spring engagement position and the resting position is equal to the gap between the second set of stiff springs 350 and the forward facing abutment 154 when the anvil system 200 is in the resting position.

Due to the central range of motion over which the stiff springs 320, 350 do not engage, the stiff springs 320, 350 do not reduce the force imparted by the anvil system 200 over this range. Accordingly, the stiff springs 320, 350 do not limit the initial impact force provided by the impactor 100. Having said this, once the anvil system 200 has travelled beyond this initial range of motion, the stiff springs 320, 350 engage to help stop the anvil system 200 and help urge the anvil system 200 back towards its resting position.

The weak springs 310, 340 bias the anvil system 200 over a range of motion that extends over at least a portion of the range between the first and second stiff spring engagement positions. For instance, the first weak spring 310 biases the anvil system 200 over a range of motion that encompasses at least a portion of a range between the first stiff spring engagement position and the resting position. Similarly, the second weak spring 340 biases the anvil system 200 over a range of motion that encompasses at least a portion of a range between the second stiff spring engagement position and the resting position.

In the present embodiment, each of the weak springs 310, 340 biases the anvil system 200 over a range of motion that ends at the resting position of the anvil system 200, but this is not essential, provided that each of the weak springs 310, 340 engage the anvil system 200 before (e.g. at a lower displacement than) the stiff springs 320, 350. This means that the weak springs 310, 340 can help to centre the anvil system 200 (and, by extension, the striker 500) after the stiff springs 320, 350 disengage. Similarly, whilst the weak springs 310, 340 bias the anvil system 200 over a range of motion that ends at the first and second end points, this also is not essential provided that the weak springs 310, 340 provide biasing forces over at least a portion of the central range (between the first and second stiff spring engagement positions).

In addition to the weak and stiff springs, dampers 330, 360 are also provided to provide end stops. The dampers 330, 360 are configured to prevent the anvil system 200 from travelling beyond the first or second end points. The dampers 330, 360 may be made of shock-absorbent material, such as rubber or silicone. The dampers 330, 360 can be configured to cushion impacts between the anvil system 200 and housing 120 to dampen vibrations.

As an alternative to dampers 330, 360, a further set of springs (or other type of resilient member) may be used, provided that they have a higher stiffness (or at least provide a stronger biasing force) than the stiff springs 320, 350.

A first damper 330 (e.g. a set of one or more third resilient members) is positioned to provide a rearward directed biasing force in response to the anvil system 200 being displaced forward beyond a first damper engagement position (e.g. a third engagement position). The first damper engagement position is located between the first stiff spring engagement position and the first end point. That is, the first damper 330 engages the anvil system 200 at a further displacement than the first set of stiff springs 320. The first damper 330 provides a biasing force over a range of motion between the first damper engagement position and the first end point.

A second damper 360 (e.g. a set of one or more sixth resilient members) is positioned to provide a forward directed biasing force in response to the anvil system 200 being displaced rearward beyond a second damper engagement position (e.g. a sixth engagement position). The second damper engagement position is located between the second stiff spring engagement position and the second end point. That is, the second damper 360 engages the anvil system 200 at a further displacement than the second set of stiff springs 350. The second damper 360 provides a biasing force over a range of motion between the second damper engagement position and the second end point.

The first damper 330 and the second damper 360 may each be considered a resilient member. Each damper 330, 360 is configured to resist movement beyond their respective engagement positions. In one embodiment, one or both of the dampers 330, 360 are made of a compressible material, such as rubber or silicone. One or both of the dampers 330, 360 may have a stiffness that is greater than that of the stiff springs 320, 350; although this is not essential. In an alternative embodiment, one or both of the dampers 330, 360 are configured to resist motion through a biasing force that is proportional to the velocity of the respective anvil 210, 220 (e.g. one or both of the dampers 330, 360 may comprise a dashpot). One or both of the dampers 330, 360 may make use of viscous friction to oppose movement.

In the present embodiment, both the dampers 330, 360 act on the rear anvil 220. This is not essential, however, and one or both of the dampers 330, 360 may act on the front anvil 210 or on another portion of the anvil system 200.

In the present embodiment, the dampers 330, 360 are ring shaped. This is not essential, however, and alternative shapes, numbers and arrangements of dampers may be used.

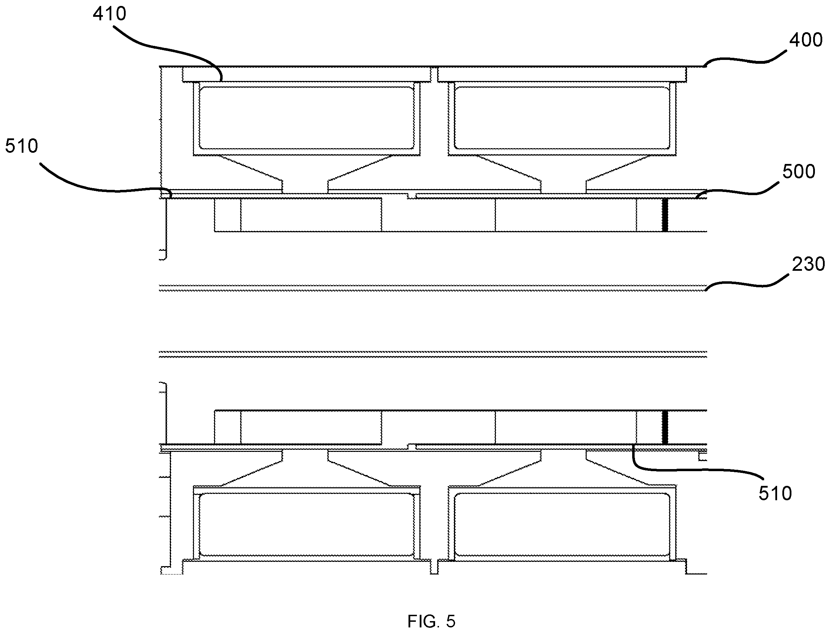

FIG. 5 shows a zoomed in cross-section of a central portion of the driving mechanism. The motor 400 surrounds the cavity within which the striker 500 is positioned. The striker 500 includes a cylindrical body 510 with a central cavity through which the connector 230 passes. The striker 500 is configured to slide forwards and rearwards along a longitudinal axis defined by the connector 230.

The striker 500 includes magnets 520 configured to be driven by the motor 400. In the present embodiment, the magnets 520 are annular (ring shaped) and are radially magnetised. In the present embodiment, two ring-shaped magnets 520 are provided; however, differing numbers of magnets 520 (e.g. four magnets) may be used. Furthermore, different shapes of magnets 520 may be used.

The motor 400 comprises a number of electromagnets 410. In the present case, the motor 400 is a rectilinear motor configured to drive the striker 500 forwards and rearwards along the longitudinal axis. A driving force may be provided by the motor 400 through adjusting the current passing through each electromagnet 410 to provide a moving magnetic field. The force provided by the motor 400 may be controlled by adjusting the magnitude and rate of change of the currents. In the present embodiment, two electromagnets 410 are provided; however, differing numbers of electromagnets 410 (e.g. four electromagnets) may be used. The motor 400 may include the same number of electromagnets 410 as the number of magnets 520 in the striker 500.

Processing circuitry (not shown) is configured to control the motor 400. The processing circuitry is configured to control the motor 400 to drive the striker 500 in response to an input from the user through the trigger 140.

The motor 400 can be controlled to drive the striker 500 to provide a single impact, or a sequence of impacts. Once the user engages the trigger 140, the striker 500 is driven to impact the anvil system 200 once. If the user continues to engage (hold down) the trigger 140, then the processing circuitry controls the motor 400 to reciprocate the striker 500 to provide a continuous sequence of impacts. At the point that the user releases the trigger 140, then the motor 400 disengages and the striker 500 is returned to its resting position by the centring system 300.

The processing circuitry is configured to control the motor 400 according to one of three selectable modes of operation: forward mode, reverse mode, and reciprocating mode. The processing circuitry is configured to switch between these modes of operation based on an input from the user through the mode switch 144.

In forward mode, the impactor 100 is configured to provide a primary force in the forward direction. In reverse mode, the impactor 100 is configured to provide a primary force in the rearward direction. In reciprocating mode, the impactor 100 is configured to alternately provide forward and backward primary forces. The strength of the primary force(s) imparted by the impactor 100 can be set through input from the user via the power control dial 142.

When driven (e.g. in response to an input via the trigger 140), the motor 400 may initiate the striker 500 by moving the striker 500 to an initiation position. In forward mode, the initiation position may be rearward of the resting position of the striker 500. In reverse mode, the initiation position may be forwards of the resting position of the striker 500. In reciprocating mode, the initiation position may depend on whether the first primary impact is to be a forward or rearward impact. In certain embodiments, the first primary impact in reciprocating mode is a forwards impact. Positioning the striker 500 at the initiation position provides the striker 500 with an increased distance over which it can be accelerated towards the anvil system 200.

The initiation movement may result in the striker 500 contacting the anvil system 200. In addition, this initiation movement may result in the striker 500 moving the anvil system 200. For instance, when initiating ahead of a forward drive (e.g. in forward mode) the striker 500 may contact the rear anvil 220. When initiating ahead of a rearward drive (e.g. in reverse mode) the striker 500 may contact the forward anvil 210. Whilst this initiation movement may provide a force (e.g. a secondary force) on the anvil system (that is opposite to the primary force), this secondary force is less than the primary force that is imparted when the striker 500 is driven. Accordingly, in forward mode, a secondary force may be imparted rearwards before the striker 500 is driven forwards to provide a primary force forwards. Similarly, in reverse mode, a secondary force may be imparted forwards before the striker 500 is driven rearwards to provide a primary force rearwards.

When initiating ahead of a forward drive, the initiation position may move the front anvil 210 to begin touching the second set of stiff springs 350 against the forward facing abutment 154 (e.g. move the front anvil 210 to the second stiff spring engagement position). When initiating ahead of a rearward drive, the initiation position may move the rear anvil 230 to begin touching the first set of stiff springs 320 to the rearward facing abutment 152 (e.g. move the rear anvil 220 to the first stiff spring engagement position).

After initiation, the striker 500 is driven to impact the anvil system 200 to impart the primary force (e.g. via a tool secured in the tool holder 110). In forward mode the striker 500 is driven forwards to impact the front anvil 210 and impart a primary force in the forward direction. In reverse mode the striker 500 is driven forwards to impact the rear anvil 220 and impart a primary force in the rearward direction. After being driven, the centring system 300 biases the striker 500 (through the anvil system 200) towards a central position (e.g. the resting position of the striker 500), and assists in moving the striker 500 back towards the central position. This can allow the striker 500 to be driven outside of the normal range of operation of the motor 400, but still be returned.

The normal range of operation of the motor 400 may be a range of positions of the striker 500 over which the motor 400 can maintain control of the striker 500. Outside of this normal range of operation, the motor 400 may be unable to control the striker 500 (e.g. to retract the striker 500) without an external assistance (such as from the centring system 300). Similarly, outside the normal range of operation, the motor 400 and striker 500 may jam (e.g. due to cogging) without external assistance (such as from the centring system 300).

Once the striker 500 has been returned to within the normal range or operation, the motor 400 may engage the striker 500 to move the striker back to its resting position, although in alternative embodiments, this may be achieved through use of the centring system 300 alone.

If the striker 500 is being driven again (e.g. if the user continues to engage the trigger 140) during forward or reverse mode then the motor 400 may move the striker 500 back to the initiation position before driving the striker 400 to impact the anvil system 200 again.

In reciprocating mode, the striker 500 may be moved to the initiation position ahead of the first impact in a similar manner to forward or reverse mode (depending on the direction of the first impact), but after this point the driving process may differ. Instead of using the motor 400 to move the striker 500 to the initiation position, the striker 500 may instead be driven in the opposite direction to produce an impact with a primary force in the opposite direction. As the direction of drive alternates, the centring system 300 may position the striker 500 and anvil system 200 in the initiation position for the next drive. For instance, after a forward impact, the striker 500 may be driven to impact the rear anvil 220 to provide a rearward impact. Similarly, after a rearward impact, the striker 500 may be driven to impact the forward anvil 210 to provide a forward impact. In this manner, the anvil system 300 may be reciprocated to provide alternating forward and rearward impact forces. As the range of motion of the striker 500 is greater than the distance between the forward anvil 210 and rear anvil 220, the striker 500 may be reciprocated to alternately impact the front anvil 210 and rear anvil 220. This process can continue until the user releases the trigger 140. In one particular embodiment, the motor 400 is configured to drive the striker over a displacement range of ± 9 mm and the first and second anvils are separated from each other by 16 mm, although alternative arrangements are possible.

Reciprocating mode can be useful when performing broaching. In a broaching process, a broaching tool (a broach) is driven into a channel and then is pulled out of the channel. As the tool is pulled out of the channel, debris that has been built up within the channel is pulled out of the channel. This frees up space in the channel for the broach to be further inserted into the channel. By removing debris from the channel, radial forces against the walls of the channel are reduced, thereby reducing the chance of breakage of the material being broached. Broaching may be used to increase the size of a channel or cavity within a bone. For instance, in femoral broaching (e.g. in total hip arthroplasty) a channel is formed in the medullary canal to receiving a femoral stem portion of a hip implant. By reciprocating the broaching tool, debris is cleared from the channel, thereby reducing the risk of radial splitting of the femur during the broaching process.

Forward mode can be used to hammer an object. For instance, an implant may be hammered into a channel or fitting through the use of a hammer attachment. Reverse mode can be used to pull an object. For instance, a pulling attachment may be connected to, secured to, engage with, or grip an object, and the reverse mode can be used to pull the object (e.g. pull the object out of another object). For instance, reverse mode can be used to pull (or extract) an implant from a channel or fitting in which the implant is fitted.

The above description refers to a "resting position" of the anvil system 200. It will be appreciated that as the first anvil 210 and the second anvil 220 are separated from each other, they may have different respective resting positions.

The use of the term "resting position" this generally refers to a position particular component (e.g. the anvil system 200) when not receiving any external forces. It should be noted that position of the anvil system 200 may move depending on external forces being applied to the anvil system 200. For instance, if the impactor 100 is being pushed against an object, a rearward force may be applied to the anvil system 200. This may move the anvil system 200 rearwards within the housing 120. Similarly, if the impactor 100 is being pulled away from an object (e.g. through the use of a pulling tool), a forward force may be applied to the anvil system 200. This may move the anvil system 200 forwards within the housing 120. Moving the position of the anvil system 200 through the application of external forces (e.g. pushing/pulling the impactor 100) can alter the forces applied by the impactor 100, by adjusting the relative positions of the anvil system 200, striker 500 and centring system 300.

The embodiments described herein provide an impactor 100 that includes a centring system 300 that allows a striker 500 to be electromagnetically driven by a motor 400 over a larger range of motion without jamming. A centring system 300 is provided that acts on an anvil system 200 against which the striker 500 is driven. As the centring system 300 acts on the anvil system 200, rather than acting on the striker 500 directly, the centring system 300 allows the striker 500 to be returned to a central position without negatively impacting the initial impact force conveyed between the striker 500 and anvil system 200. As the centring system 300 includes resilient members (e.g. springs and dampers) that have increasing stiffness and engage at increasing displacements, the striker 500 can be effectively centred and its movement can be arrested with a reduced effect on the initial impact force of the impactor 100.

Particular embodiments include a forward anvil 210 and a rear anvil 220, allowing forward and reverse forces to be imparted by the impactor 100. The range of motion of the striker 500 may be greater than the distance between the front anvil 210 and the rear anvil 220. This allows reciprocating operation to be provided through alternately impacting the forward anvil 210 and the rear anvil 220.

Whilst the description includes discussion of "forward" and "rearward" directions, it will be appreciated that these directions are relative to each other and to the arrangement of the impactor 100, and to not imply any particular orientation of the impactor 100 in use. More generally, the forward direction can be considered a first direction along a longitudinal axis of the impactor 100. The second direction can be considered a second direction along the longitudinal axis of the impactor 100, wherein the second direction is opposite to the first direction. Similarly, the forward anvil 210 may be considered a first anvil for imparting a force in the first direction and the rearward anvil 220 may be considered a second anvil for imparting a force in the second direction.

The embodiments described herein can include processing circuitry for controlling the operation of the impactor 100. This can be realized in digital electronic circuitry, in computer software, firmware, and/or hardware. Examples of hardware elements may include processors, microprocessors, circuits, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth.

While certain arrangements have been described, the arrangements have been presented by way of example only, and are not intended to limit the scope of protection. The inventive concepts described herein may be implemented in a variety of other forms. In addition, various omissions, substitutions and changes to the specific implementations described herein may be made without departing from the scope of protection defined in the following claims.

## Claims

1. An electromagnetic surgical impactor (100) comprising:
a housing (120) forming an internal cavity extending along a longitudinal axis;
a striker (500) located within the internal cavity and configured to move along the longitudinal axis;
an anvil system (200) comprising a first anvil (210) wherein the anvil system (200) is configured to move in a first direction along the longitudinal axis from a first resting position towards a first end point,
an electromagnetic drive system (400) configured to drive the striker (500) to move in the first direction to impact the first anvil (210) such that a first force directed in the first direction is imparted to an external object via the first anvil (210); and
a centring system (300) configured to bias the first anvil (210) towards the first resting position, wherein the centring system (300) comprises:
one or more first resilient members (310) configured to bias the first anvil (210) towards the first resting position in response to the first anvil (210) being displaced towards the first end point, beyond a first engagement position; and
one or more second resilient members (320) configured to bias the first anvil (210) towards the first resting position in response to the first anvil (210) being displaced towards the first end point, beyond a second engagement position, wherein the second engagement position is located between the first engagement position and the first end point,
**characterised in that**
each of the first resilient members (310) has a lower stiffness than each of the second resilient members (320).

2. The electromagnetic surgical impactor (100) of claim 1 wherein the first and second resilient members are springs, wherein each second resilient member (320) has a higher spring constant than each first resilient member (310).

3. The electromagnetic surgical impactor of claim 2 wherein the one or more first resilient members comprise one or more coil springs and/or wherein the one or more second resilient members comprise one or more leaf springs.

4. The electromagnetic surgical impactor (100) of any preceding claim wherein:
the centring system (300) further includes one or more third resilient members (330) configured to bias the first anvil (210) towards the first resting position in response to the first anvil (210) being displaced towards the first end point, beyond a third engagement position, wherein the third engagement position is located between the second engagement position and the first end point.

5. The electromagnetic surgical impactor of claim 4 wherein:
the one or more third resilient members comprises one or more dampers, each comprising a shock absorbent material; and/or
the centring system (300) is configured to prevent the striker (500) travelling beyond the first end point.

6. The electromagnetic surgical impactor (100) of any preceding claim wherein:
the anvil system (200) comprises a second anvil (220) connected to the first anvil (210), wherein the second anvil (220) is configured to move in a second direction, opposite to the first direction, from a second resting position to a second end point, wherein the first and second anvils are positioned on opposite sides of the striker (500);
the electromagnetic drive system (400) is further configured to drive the striker (500) to move in the second direction to impact the second anvil (220) such that a second force directed in the second direction is imparted to the external object via the second anvil (220); and
the centring system (300) further comprises:
one or more fourth resilient members (340) configured to bias the second anvil (220) towards the second resting position in response to the second anvil (220) being displaced towards the second end point, beyond a fourth engagement position; and
one or more fifth resilient members (350) configured to bias the second anvil (220) towards the second resting position in response to the second anvil (220) being displaced towards the second end point, beyond a fifth engagement position, wherein the fifth engagement position is located between the fourth engagement position and the second end point,
wherein each of the fourth resilient members (340) has a lower stiffness than each of the fifth resilient members (350).

7. The electromagnetic surgical impactor (100) of claim 6 wherein the fourth and fifth resilient members are springs, wherein each fifth resilient member (350) has a higher spring constant than each fourth resilient member (340).

8. The electromagnetic surgical impactor of claim 7 wherein the one or more fourth resilient members comprise one or more coil springs and/or wherein the one or more fifth resilient members comprise one or more leaf springs.

9. The electromagnetic surgical impactor (100) of any of claims 6-8 wherein the centring system (300) further includes one or more sixth resilient members (360) configured to bias the second anvil (220) towards the second resting position in response to the second anvil (220) being displaced towards the second end point, beyond a sixth engagement position, wherein the sixth engagement position is located between the fifth engagement position and the second end point.

10. The electromagnetic surgical impactor of claim 9 wherein the one or more sixth resilient members comprises one or more dampers, each comprising a shock absorbent material.

11. The electromagnetic surgical impactor (100) of any of claims 6-10 wherein the centring system (300) is configured to prevent the striker (500) travelling beyond the second end point.

12. The electromagnetic surgical impactor (100) of any of claims 6-11 wherein the separation between the first and second anvils in the first direction is less than a maximum range of motion over which the electromagnetic drive system (400) is configured to drive the striker (500).

13. The electromagnetic surgical impactor (100) of any of claims 6-12 further comprising circuitry (144) configured to control the electromagnetic drive system (400) to drive the striker (500) according to a mode of operation, wherein:
the first anvil (210) is connected to the second anvil (220) via a rod which passes through a channel in the striker (500); and/or
the mode of operation is selectable between:
a first mode of operation that controls the electromagnetic drive system (400) to drive the striker (500) in the first direction to impact the first anvil (210) to impart a primary force in the first direction;
a second mode of operation that controls the electromagnetic drive system (400) to drive the striker (500) in the second direction to impact the second anvil (220) to impart a primary force in the second direction; and
a reciprocating mode of operation that controls the electromagnetic drive system (400) to alternately drive the striker (500) in the first direction and in the second direction to alternately impact the first anvil (210) to impart a primary force in the first direction and impact the second anvil (220) to impart a primary force in the second direction.

14. The electromagnetic surgical impactor (100) of any of claims 6-13 wherein:
at least one of the one or more first resilient members (310) and the one or more second resilient members is configured to impart a first biasing force on the second anvil (220) in the second direction to impart tension on the anvil system (200) when biasing the first anvil (210) towards the first resting position; and/or
at least one of the one or more fourth resilient members (340) and the one or more fifth resilient members (350) is configured to impart a second biasing force on the first anvil (210) in the first direction to impart tension on the anvil system (200) when biasing the second anvil (220) towards the second resting position.

15. The electromagnetic surgical impactor (100) of any preceding claim wherein;
the centring system (300) is configured to impart a biasing force to the striker (500) via the anvil system (200) to bias the striker (500) towards a central position after each impact between the striker (500) and the anvil system (200); and/or
the electromagnetic surgical impactor (100) further comprises a tool or tool holder (110) connected to the second anvil (220) for imparting force from the second anvil (220) to the external object; and/or
the centring system (300) is configured to allow the anvil system (200) to float within the housing (120) to allow a user to alter an impact force between the striker (500) and the anvil system (200) through pushing or pulling the electromagnetic surgical impactor (100) to adjust a position of the anvil system (200) relative to the striker (500).

## Patentansprüche

1. Elektromagnetischer chirurgischer Impaktor (100), umfassend:
ein Gehäuse (120), das einen sich entlang einer Längsachse erstreckenden internen Hohlraum bildet;
ein Schlaggerät (500), das sich innerhalb des internen Hohlraums befindet und konfiguriert ist, um sich entlang der Längsachse zu bewegen;
ein Ambosssystem (200), umfassend einen ersten Amboss (210), wobei das Ambosssystem (200) konfiguriert ist, um sich entlang der Längsachse in einer ersten Richtung von einer ersten Ruheposition hin zu einem ersten Endpunkt zu bewegen,
ein elektromagnetisches Antriebssystem (400), das konfiguriert ist, um das Schlaggerät (500) anzutreiben, um sich in die erste Richtung zu bewegen, um derart auf den ersten Amboss (210) aufzutreffen, dass eine erste in die erste Richtung gerichtete Kraft mittels des ersten Ambosses (210) auf ein externes Objekt übertragen wird; und
ein Zentriersystem (300), das konfiguriert ist, um den ersten Amboss (210) zu der ersten Ruheposition hin vorzuspannen, wobei das Zentriersystem (300) Folgendes umfasst:
ein oder mehrere erste elastische Elemente (310), die konfiguriert sind, um als Reaktion darauf, dass der erste Amboss (210) über eine erste Eingriffsposition hinaus zu dem ersten Endpunkt hin verschoben wird, den ersten Amboss (210) zu der ersten Ruheposition hin vorzuspannen; und
ein oder mehrere zweite elastische Elemente (320), die konfiguriert sind, um als Reaktion darauf, dass der erste Amboss (210) über eine zweite Eingriffsposition hinaus zu dem ersten Endpunkt hin verschoben wird, den ersten Amboss (210) zu der ersten Ruheposition hin vorzuspannen, wobei sich die zweite Eingriffsposition zwischen der ersten Eingriffsposition und dem ersten Endpunkt befindet,
**dadurch gekennzeichnet, dass**
jedes der ersten elastischen Elemente (310) eine geringere Steifigkeit als jedes der zweiten elastischen Elemente (320) aufweist.

2. Elektromagnetischer chirurgischer Impaktor (100) nach Anspruch 1, wobei die ersten und die zweiten elastischen Elemente Federn sind, wobei jedes zweite elastische Element (320) eine höhere Federkonstante als jedes erste elastische Element (310) aufweist.

3. Elektromagnetischer chirurgischer Impaktor nach Anspruch 2, wobei das eine oder die mehreren ersten elastischen Elemente eine oder mehrere Schraubenfedern umfassen und/oder wobei das eine oder die mehreren zweiten elastischen Elemente eine oder mehrere Blattfedern umfassen.

4. Elektromagnetischer chirurgischer Impaktor (100) nach einem vorstehenden Anspruch, wobei:
das Zentriersystem (300) weiter ein oder mehrere dritte elastische Elemente (330) einschließt, die konfiguriert sind, um als Reaktion darauf, dass der erste Amboss (210) über eine dritte Eingriffsposition hinaus zu dem ersten Endpunkt hin verschoben wird, den ersten Amboss (210) zu der ersten Ruheposition hin vorzuspannen, wobei sich die dritte Eingriffsposition zwischen der zweiten Eingriffsposition und dem ersten Endpunkt befindet.

5. Elektromagnetischer chirurgischer Impaktor nach Anspruch 4, wobei:
das eine oder die mehreren elastischen Elemente einen oder mehrere Dämpfer umfassen, die jeweils ein stoßdämpfendes Material umfassen; und/oder
das Zentriersystem (300) konfiguriert ist, um das Schlaggerät (500) daran zu hindern, über den ersten Endpunkt hinauszufahren.

6. Elektromagnetischer chirurgischer Impaktor (100) nach einem vorstehenden Anspruch, wobei:
das Ambosssystem (200) einen zweiten Amboss (220) umfasst, der mit dem ersten Amboss (210) verbunden ist, wobei der zweite Amboss (220) konfiguriert ist, um sich in einer zweiten Richtung, entgegengesetzt zu der ersten Richtung, von einer zweiten Ruheposition zu einem zweiten Endpunkt zu bewegen, wobei der erste und der zweite Amboss auf gegenüberliegenden Seiten des Schlaggeräts (500) positioniert sind;
das elektromagnetische Antriebssystem (400) weiter konfiguriert ist, um das Schlaggerät (500) in die zweite Richtung zu bewegen, um auf den zweiten Amboss (220) derart aufzutreffen, dass eine zweite in die zweite Richtung gerichtete Kraft mittels des zweiten Ambosses (220) auf das externe Objekt übertragen wird; und
das Zentriersystem (300) weiter umfasst:
ein oder mehrere vierte elastische Elemente (340), die konfiguriert sind, um als Reaktion darauf, dass der zweite Amboss (220) über eine vierte Eingriffsposition hinaus zu dem zweiten Endpunkt hin verschoben wird, den zweiten Amboss (220) zu der zweiten Ruheposition hin vorzuspannen; und
ein oder mehrere fünfte elastische Elemente (350), die konfiguriert sind, um als Reaktion darauf, dass der zweite Amboss (220) über eine fünfte Eingriffsposition hinaus zu dem zweiten Endpunkt hin verschoben wird, den zweiten Amboss (220) zu der zweiten Ruheposition hin vorzuspannen, wobei sich die fünfte Eingriffsposition zwischen der vierten Eingriffsposition und dem zweiten Endpunkt befindet,
wobei jedes der vierten elastischen Elemente (340) eine geringere Steifigkeit als jedes der fünften elastischen Elemente (350) aufweist.

7. Elektromagnetischer chirurgischer Impaktor (100) nach Anspruch 6, wobei die vierten und fünften elastischen Elemente Federn sind, wobei jedes fünfte elastische Element (350) eine höhere Federkonstante als jedes vierte elastische Element (340) aufweist.

8. Elektromagnetischer chirurgischer Impaktor nach Anspruch 7, wobei das eine oder die mehreren vierten elastischen Elemente eine oder mehrere Schraubenfedern umfassen und/oder wobei das eine oder die mehreren fünften elastischen Elemente eine oder mehrere Blattfedern umfassen.

9. Elektromagnetischer chirurgischer Impaktor (100) nach einem der Ansprüche 6-8, wobei das Zentriersystem (300) weiter ein oder mehrere sechste elastische Elemente (360) einschließt, die konfiguriert sind, um als Reaktion darauf, dass der zweite Amboss (220) über eine sechste Eingriffsposition hinaus zu dem zweiten Endpunkt hin verschoben wird, den zweiten Amboss (220) zu der zweiten Ruheposition hin vorzuspannen, wobei sich die sechste Eingriffsposition zwischen der fünften Eingriffsposition und dem zweiten Endpunkt befindet.

10. Elektromagnetischer chirurgischer Impaktor nach Anspruch 9, wobei das eine oder die mehreren sechsten elastischen Elemente einen oder mehrere Dämpfer umfasst, die jeweils ein stoßdämpfendes Material umfassen.

11. Elektromagnetischer chirurgischer Impaktor (100) nach einem der Ansprüche 6-10, wobei das Zentriersystem (300) konfiguriert ist, um das Schlaggerät (500) daran zu hindern, über den zweiten Endpunkt hinauszufahren.

12. Elektromagnetischer chirurgischer Impaktor (100) nach einem der Ansprüche 6-11, wobei der Abstand zwischen dem ersten und dem zweiten Amboss in der ersten Richtung kleiner als ein maximaler Bewegungsbereich ist, über den das elektromagnetische Antriebssystem (400) konfiguriert ist, um das Schlaggerät (500) anzutreiben.

13. Elektromagnetischer chirurgischer Impaktor (100) nach einem der Ansprüche 6-12, weiter umfassend eine Schaltung (144), die konfiguriert ist, um das elektromagnetische Antriebssystem (400) zu steuern, um das Schlaggerät (500) gemäß einem Betriebsmodus anzutreiben, wobei:
der erste Amboss (210) mittels einer Stange, die einen Kanal in dem Schlaggerät (500) durchquert, mit dem zweiten Amboss (220) verbunden ist; und/oder
der Betriebsmodus auswählbar ist zwischen:
einem ersten Betriebsmodus, der das elektromagnetische Antriebssystem (400) steuert, um das Schlaggerät (500) in die erste Richtung anzutreiben, um auf den ersten Amboss (210) aufzutreffen, um eine Primärkraft in die erste Richtung zu übertragen;
einem zweiten Betriebsmodus, der das elektromagnetische Antriebssystem (400) steuert, um das Schlaggerät (500) in die zweite Richtung anzutreiben, um auf den zweiten Amboss (220) aufzutreffen, um eine Primärkraft in die zweite Richtung zu übertragen; und
einem oszillierenden Betriebsmodus, der das elektromagnetische Antriebssystem (400) steuert, um das Schlaggerät (500) abwechselnd in die erste Richtung und in die zweite Richtung anzutreiben, um abwechselnd auf den ersten Amboss (210) aufzutreffen, um eine Primärkraft in die erste Richtung zu übertragen, und auf den zweiten Amboss (220) aufzutreffen, um eine Primärkraft in die zweite Richtung zu übertragen.

14. Elektromagnetischer chirurgischer Impaktor (100) nach einem der Ansprüche 6-13, wobei:
mindestens eines des einen oder der mehreren ersten elastischen Elemente (310) und des einen oder der mehreren zweiten elastischen Elemente konfiguriert ist, um eine erste Vorspannkraft auf den zweiten Amboss (220) in der zweiten Richtung zu übertragen, um eine Spannung auf das Ambosssystem (200) zu übertragen, wenn der erste Amboss (210) zu der ersten Ruheposition hin vorgespannt wird; und/oder
mindestens eines des einen oder der mehreren vierten elastischen Elemente (340) und des einen oder der mehreren fünften elastischen Elemente (350) konfiguriert ist, um eine zweite Vorspannkraft auf den ersten Amboss (210) in der ersten Richtung zu übertragen, um eine Spannung auf das Ambosssystem (200) zu übertragen, wenn der zweite Amboss (220) zu der zweiten Ruheposition hin vorgespannt wird.

15. Elektromagnetischer chirurgischer Impaktor (100) nach einem vorstehenden Anspruch, wobei;
das Zentriersystem (300) konfiguriert ist, um mittels des Ambosssystems (200) eine Vorspannkraft auf das Schlaggerät (500) zu übertragen, um das Schlaggerät (500) nach jedem Aufprall zwischen dem Schlaggerät (500) und dem Ambosssystem (200) zu einer zentralen Position hin vorzuspannen; und/oder
der elektromagnetische chirurgische Impaktor (100) weiter ein Werkzeug oder einen Werkzeughalter (110) umfasst, der mit dem zweiten Amboss (220) verbunden ist, um Kraft von dem zweiten Amboss (220) auf das externe Objekt zu übertragen; und/oder
das Zentriersystem (300) konfiguriert ist, um dem Ambosssystem (200) zu erlauben, innerhalb des Gehäuses (120) zu gleiten, um einem Benutzer zu erlauben, die Aufprallkraft zwischen dem Schlaggerät (500) und dem Ambosssystem (200) durch Drücken oder Ziehen des elektromagnetischen chirurgischen Impaktors (100) zu verändern, um eine Position des Ambosssystems (200) relativ zu dem Schlaggerät (500) anzupassen.

## Revendications

1. Impacteur chirurgical électromagnétique (100) comprenant :
un boîtier (120) formant une cavité interne s'étendant le long d'un axe longitudinal ;
un percuteur (500) situé dans la cavité interne et configuré pour se déplacer le long de l'axe longitudinal ;
un système d'enclume (200) comprenant une première enclume (210), dans lequel le système d'enclume (200) est configuré pour se déplacer dans une première direction le long de l'axe longitudinal à partir d'une première position de repos vers un premier point d'extrémité,
un système d'entraînement électromagnétique (400) configuré pour entraîner le percuteur (500) à se déplacer dans la première direction afin de percuter la première enclume (210) de sorte qu'une première force dirigée dans la première direction soit transmise à un objet externe via la première enclume (210) ; et
un système de centrage (300) configuré pour orienter la première enclume (210) vers la première position de repos, dans lequel le système de centrage (300) comprend :
un ou plusieurs premiers éléments élastiques (310) configurés pour orienter la première enclume (210) vers la première position de repos en réponse au déplacement de la première enclume (210) vers le premier point d'extrémité, au-delà d'une première position de mise en prise ; et
un ou plusieurs deuxièmes éléments élastiques (320) configurés pour orienter la première enclume (210) vers la première position de repos en réponse au déplacement de la première enclume (210) vers le premier point d'extrémité, au-delà d'une deuxième position de mise en prise, dans lequel la deuxième position de mise en prise est située entre la première position de mise en prise et le premier point d'extrémité,
**caractérisé en ce que**
chacun des premiers éléments élastiques (310) présente une rigidité inférieure à celle de chacun des deuxièmes éléments élastiques (320).

2. Impacteur chirurgical électromagnétique (100) selon la revendication 1 dans lequel les premier et deuxième éléments élastiques sont des ressorts, dans lequel chaque deuxième élément élastique (320) présente une constante de raideur plus élevée que chaque premier élément élastique (310).

3. Impacteur chirurgical électromagnétique selon la revendication 2, dans lequel les un ou plusieurs premiers éléments élastiques comprennent un ou plusieurs ressorts hélicoïdaux et/ou dans lequel les un ou plusieurs deuxièmes éléments élastiques comprennent un ou plusieurs ressorts à lames.

4. Impacteur chirurgical électromagnétique (100) selon une quelconque revendication précédente dans lequel :
le système de centrage (300) inclut en outre un ou plusieurs troisièmes éléments élastiques (330) configurés pour orienter la première enclume (210) vers la première position de repos en réponse au déplacement de la première enclume (210) vers le premier point d'extrémité, au-delà d'une troisième position de mise en prise, dans lequel la troisième position de mise en prise est située entre la deuxième position de mise en prise et le premier point d'extrémité.

5. Impacteur chirurgical électromagnétique selon la revendication 4, dans lequel :
le ou les troisièmes éléments élastiques comprennent un ou plusieurs amortisseurs, chacun comprenant un matériau absorbant les chocs ; et/ou
le système de centrage (300) est configuré pour empêcher le percuteur (500) de se déplacer au-delà du premier point d'extrémité.

6. Impacteur chirurgical électromagnétique (100) selon une quelconque revendication précédente dans lequel :
le système d'enclume (200) comprend une deuxième enclume (220) reliée à la première enclume (210), dans lequel la deuxième enclume (220) est configurée pour se déplacer dans une deuxième direction, opposée à la première direction, d'une deuxième position de repos à un deuxième point d'extrémité, dans lequel la première et la deuxième enclume sont positionnées de part et d'autre du percuteur (500) ;
le système d'entraînement électromagnétique (400) est en outre configuré pour entraîner le percuteur (500) à se déplacer dans la deuxième direction afin de percuter la deuxième enclume (220) de sorte qu'une deuxième force dirigée dans la deuxième direction soit transmise à l'objet externe via la deuxième enclume (220) ; et
le système de centrage (300) comprend en outre :
un ou plusieurs quatrièmes éléments élastiques (340) configurés pour orienter la deuxième enclume (220) vers la deuxième position de repos en réponse au déplacement de la deuxième enclume (220) vers le deuxième point d'extrémité, au-delà d'une quatrième position de mise en prise ; et
un ou plusieurs cinquièmes éléments élastiques (350) configurés pour orienter la deuxième enclume (220) vers la deuxième position de repos en réponse au déplacement de la deuxième enclume (220) vers le deuxième point d'extrémité, au-delà d'une cinquième position de mise en prise, dans lequel la cinquième position de mise en prise est située entre la quatrième position de mise en prise et le deuxième point d'extrémité,
dans lequel chacun des quatrièmes éléments élastiques (340) présente une rigidité inférieure à celle de chacun des cinquièmes éléments élastiques (350).

7. Impacteur chirurgical électromagnétique (100) selon la revendication 6 dans lequel les quatrième et cinquième éléments élastiques sont des ressorts, dans lequel chaque cinquième élément élastique (350) présente une constante de raideur plus élevée que chaque quatrième élément élastique (340).

8. Impacteur chirurgical électromagnétique selon la revendication 7, dans lequel les un ou plusieurs quatrièmes éléments élastiques comprennent un ou plusieurs ressorts hélicoïdaux et/ou dans lequel les un ou plusieurs cinquièmes éléments élastiques comprennent un ou plusieurs ressorts à lames.

9. Impacteur chirurgical électromagnétique (100) selon l'une quelconque des revendications 6-8, dans lequel le système de centrage (300) inclut en outre un ou plusieurs sixièmes éléments élastiques (360) configurés pour solliciter la deuxième enclume (220) vers la deuxième position de repos en réponse au déplacement de la deuxième enclume (220) vers le deuxième point d'extrémité, au-delà d'une sixième position de mise en prise, dans lequel la sixième position de mise en prise est située entre la cinquième position de mise en prise et le deuxième point d'extrémité.

10. Impacteur chirurgical électromagnétique selon la revendication 9, dans lequel les un ou plusieurs sixièmes éléments élastiques comprennent un ou plusieurs amortisseurs, chacun comprenant un matériau absorbant les chocs.

11. Impacteur chirurgical électromagnétique (100) selon l'une quelconque des revendications 6-10, dans lequel le système de centrage (300) est configuré pour empêcher le percuteur (500) de se déplacer au-delà du deuxième point d'extrémité.

12. Impacteur chirurgical électromagnétique (100) selon l'une quelconque des revendications 6-11 dans lequel la séparation entre la première et la deuxième enclume dans la première direction est inférieure à une plage de mouvement maximale sur laquelle le système d'entraînement électromagnétique (400) est configuré pour entraîner le percuteur (500).

13. Impacteur chirurgical électromagnétique (100) selon l'une quelconque des revendications 6-12 comprenant en outre un circuit (144) configuré pour commander le système d'entraînement électromagnétique (400) afin d'entraîner le percuteur (500) selon un mode de fonctionnement, dans lequel :
la première enclume (210) est reliée à la deuxième enclume (220) par le biais d'une tige qui passe à travers un canal dans le percuteur (500) ; et/ou
le mode de fonctionnement peut être sélectionné parmi les suivants :
un premier mode de fonctionnement qui commande le système d'entraînement électromagnétique (400) pour entraîner le percuteur (500) dans la première direction pour frapper la première enclume (210) afin d'imprimer une force primaire dans la première direction ;
un deuxième mode de fonctionnement qui commande le système d'entraînement électromagnétique (400) pour entraîner le percuteur (500) dans la deuxième direction afin de frapper la deuxième enclume (220) et d'appliquer une force primaire dans la deuxième direction ; et
un mode de fonctionnement alternatif qui commande le système d'entraînement électromagnétique (400) pour entraîner alternativement le percuteur (500) dans la première direction et dans la deuxième direction pour frapper alternativement la première enclume (210) afin d'imposer une force primaire dans la première direction et frapper la deuxième enclume (220) afin d'imposer une force primaire dans la deuxième direction.

14. Impacteur chirurgical électromagnétique (100) selon l'une quelconque des revendications 6-13, dans lequel :
au moins un des un ou plusieurs premiers éléments élastiques (310) et des un ou plusieurs deuxièmes éléments élastiques sont configurés pour transmettre une première force de rappel sur la deuxième enclume (220) dans la deuxième direction pour transmettre une tension sur le système d'enclume (200) lors de l'orientation de la première enclume (210) vers la première position de repos ; et/ou
au moins un des un ou plusieurs quatrièmes éléments élastiques (340) et un ou plusieurs cinquièmes éléments élastiques (350) sont configurés pour transmettre une deuxième force de rappel sur la première enclume (210) dans la première direction afin de transmettre une tension sur le système d'enclume (200) lors de l'orientation de la deuxième enclume (220) vers la deuxième position de repos.

15. Impacteur chirurgical électromagnétique (100) selon une quelconque revendication précédente dans lequel ;
le système de centrage (300) est configuré pour transmettre une force de rappel au percuteur (500) via le système d'enclume (200) afin de ramener le percuteur (500) vers une position centrale après chaque impact entre le percuteur (500) et le système d'enclume (200) ; et/ou
l'impacteur chirurgical électromagnétique (100) comprend en outre un outil ou un porte-outil (110) relié à la deuxième enclume (220) pour transmettre la force de la deuxième enclume (220) à l'objet externe ; et/ou
le système de centrage (300) est configuré pour permettre au système d'enclume (200) de flotter à l'intérieur du boîtier (120) afin de permettre à un utilisateur de modifier une force d'impact entre le percuteur (500) et le système d'enclume (200) en poussant ou en tirant l'impacteur chirurgical électromagnétique (100) afin d'ajuster la position du système d'enclume (200) par rapport au percuteur (500).
